# EUROPEAN PATENT SPECIFICATION

(11) **EP 1 315 518 B1**
(45) Date of publication and mention of the grant of the patent: **02.12.2009**
(21) Application number: 01963230.6
(22) Date of filing: 04.09.2001
(51) Int. Cl.: A61K 39/02, A61P 31/04

(54) **VACCINE AGAINST MICROBIAL PATHOGENS**
IMPSTOFF GEGEN MIKROBIELLE PATHOGENEN
VACCIN CONTRE DES MICROBES PATHOGENES

(30) Priority: 04.09.2000 GB 0021757
(43) Date of publication of application: 04.06.2003
(73) Proprietor: Immunobiology Limited, Cambridge, Cambridgeshire CB22 3AT (GB)
(72) Inventor: COLACO, Camilo, Anthony, Leo, Selwyn, Cambridge CB2 2JN (GB)
(74) Representative: Stark, Gordon Drummond
(86) International application number: PCT/GB2001/003964
(87) International publication number: WO 2002/020045

(56) References cited:
- WO-A-00/10597
- WO-A-01/13944
- WO-A-01/63278
- WO-A-96/40928
- US-A- 5 961 979

## Description

The present invention relates to a vaccine and a method for producing a vaccine. More specifically, there is provided a vaccine comprising a microbial pathogen and a method of producing the same.

### BACKGROUND OF THE INVENTION

An important component of any human immune response is the presentation of antigens to T cells by antigen presenting cells (APCs) such as macrophages, B cells or dendritic cells. Peptide fragments of foreign antigens are presented on the surface of the macrophage in combination with major histocompatibility complex (MHC) molecules, in association with helper molecules, such as CD4 and CD8 molecules. Such antigenic peptide fragments presented in this way are recognised by the T cell receptor of T cells. The interaction of the antigenic peptide fragments with the T cell receptor results in antigen-specific T cell proliferation, and secretion of lymphokines by the T-cells. The nature of the antigenic peptide fragment presented by the APCs is critical in establishing immunity.

Heat shock proteins (hsps) form a family of highly conserved proteins that are widely distributed throughout the plant and animal kingdoms. On the basis of their molecular weights, hsps are grouped into six different families: small (hsp 20-30kDa); hsp40; hsp60; hsp70; hsp90; and hsp100. Although hsps were originally identified in cells subjected to heat stress, they have been found to be associated with many other forms of stress, such as infections, and are thus also commonly referred to as stress proteins (SPs).

Members of the mammalian hsp90 family include cytosolic hsp90 (hsp83) and the endoplasmic reticulum counterparts hsp90 (hsp83), hsp87, Grp94 (Erp99) and gp97, see for example Gething et al. (1992) Nature 355:33-45. Members of the hsp70 family include cytosolic hsp70 (p73) and hsp70 (p72), the endoplasmic reticulum counterpart BiP (Grp78), and the mitochondrial counterpart hsp70 (Grp75). Members of the mammalian hsp60 family have only been identified in the mitochondria.

Stress proteins are ubiquitously expressed within cells. One of the roles of stress proteins is to chaperone peptides from one cellular compartment to another and to present peptides to the MHC molecules for cell surface presentation to the immune system. In the case of diseased cells, stress proteins also chaperone viral or tumour-associated peptides to the cell-surface, see Li and Sirivastave (1994) Behring Inst. Mitt, 94: 37-47 and Suzue et al. (1997) Proc.Natl.Acad.Sci. USA 94: 13146-51.

The chaperone function of stress proteins is accomplished through the formation of complexes between stress proteins and antigenic peptide fragments, and between stress proteins and viral or tumour-associated peptide fragments, in an ATP dependent reaction. The peptide fragments complexed with the stress proteins form antigenic complexes (hspCs) which are captured by APCs to provide antigenic peptide fragments.

The complex association of stress proteins with peptide fragments has also been observed in normal tissues and is not therefore a tumour-specific phenomenon, see Srivastava (1994) Experimentia 50: 1054-60. It is thought that the immunogenicity of the members of the hsp70 family, including grp96, reflects their normal role in the 'presentosome', the postulated intracellular organelle functioning in the loading of MHC Class I molecules required for their cell surface expression. This step is essential for their MHC-restricted recognition by antigen-specific T-cells, see Srivastava et al. (1998) Immunity, Singh-Jasuja et al. J.Exp.Med (2000) 191, 1957.

Until recently, it has not been proposed to use pathogen-derived endogenous hsp-peptide complexes (hspCs) as vaccines, despite the fact that hsps from pathogens have been used extensively as antigens and adjuvants. PCT Publication No WO 2001/013944 discloses the use of pathogen-derived endogeneous hspCs and in particular stress induced hspCs are shown to give good protective immunity in vaccinated animals.

Members of the microbial hsp family include the Dna J and Dna K families and the Gro-EL and Gro-ES families. In prokaryotic microbes it appears that these families are encoded in operons, with the initial gene in the operon being a control gene which suppresses the expression of the hsp genes contained within the operon.

In *Streptomyces* and *Helicobacter*, expression of the hspR gene suppresses the expression of Dna J and Dna K. Deletion of the hspR gene therefore results in a genetically modified microbe that constitutively expresses hsps, see Bucca et al. (1997) Mol.Microbiol 15:633-45. Homologous operons have been identified in a number of recently sequenced microbes, including other strains of *Streptomyces* and *Mycobacterium tuberculosis* and the commonly used related vaccine strain *BCG*.

Other repressor genes may also control the expression of members of the hsp gene family and that these may also be genetically engineered to provide modified microbes that constitutively expresses hspCs that may be utilised for the production of vaccines and vaccine vectors. These include, but are not limited to the transcriptional control genes sigma and rho and the stress-gene regulatory protein genes MerR and HmrR. It will also be appreciated that the modified microbes containing the constitutive hspCs may be used directly as vaccines or as a source for the isolation of the hspCs. Furthermore the expression of heterologous gene(s) from other pathogen(s) in these microbes will enable their use as vaccine vectors for the simplified production of hspC-based vaccines for these pathogens.

Whilst looking at the use of heat-induced endogeneous microbial hsp-peptide complexes, the extracted hsp-peptide complexes were compared to the use of the stressed-induced microbe itself as a vaccine. Surprisingly, the use of the microbe as a vaccine gave significantly better immunity in vaccinated animals than the use of the isolated hsp-peptide complexes. Similar results were obtained using genetically modified microbes that constitutively produced hsps, indicating that hspCs were formed in situ with endogenous microbial polypeptides, including heterogenous genes expressed as recombinant proteins in the microbe. The genetically modified microbes could also be used as an efficient source for the isolation of hspCs for use in subunit and multi-subunit vaccines.

### SUMMARY OF THE INVENTION

According to the present invention, there is provided a vaccine comprising a killed, dried or attenuated bacterial pathogen as an immunogenic determinant, wherein the bacterial pathogen has been subjected to a stress inducing stimuli wherein the stress inducing stimuli is the genetic modification of the bacterial pathogen such that at least one repressor gene for a heat shock protein gene is inactivated, thereby allowing the constitutive expression of a heat shock protein gene.

Preferably the genetic modification results in the inactivation of the hspR repressor gene.

Alternatively the genetic modification results in the inactivation of the stress gene regulatory protein genes MerR or HmrR repressor genes, or the transcriptional control genes sigma and rho.

Preferably the bacterial pathogen is selected from the group consisting of Mycobacteria, Salmonella, Vibrio, Streptomyces, Helicobacter, Lactococcus and Listeria.

Preferably the vaccine further comprises an adjuvant.

Preferably the adjuvant is selected from the group consisting of; Freund's complete adjuvant, Freund's incomplete adjuvant, Quil A, Detox, ISCOMs and squalene.

Preferably the vaccine is suitable for administration by injection.

Alternatively the vaccine is suitable for oral administration.

Preferably the vaccine is suitable for delivery by means of a needle-less delivery format.

A further aspect of the present invention provides a vaccine composition according to the present invention for use in the treatment of infection with a bacterial pathogen which is provided as the immunogenic determinant in the vaccine composition.

Preferably the vaccine is administered as a prophylactic vaccine.

Alternatively the vaccine is administered as a therapeutic vaccine.

Preferably the vaccine composition is administered by injection.

Alternatively the vaccine composition is administered by needle-less delivery.

Alternatively the vaccine composition is administered transdermally, by pulmonary delivery or orally.

A yet further aspect of the present invention provides a method of producing a vaccine composition, comprising an immunogenic determinant, **characterised in that** said method comprises the steps of; subjecting bacterial pathogens to stress inducing stimuli wherein the stress inducing stimuli is the inactivation of a gene which represses the expression of heat shock genes; and using the stressed bacterial pathogen in the preparation of the vaccine composition as said immunogenic determinant.

Preferably the repressed gene is the hspR gene.

Alternatively the repressed gene is the stress gene regulatory protein gene MerR or HmrR, or the transcriptional control genes sigma and rho.

Preferably the microbial pathogen cells are killed prior to use in said vaccine.

Alternatively the microbial pathogen cells are dried prior to use in said vaccine.

Preferably the vaccine composition is an aqueous composition.

Alternatively the vaccine composition is a dry composition or in a lyophilised composition.

Further described herein is the use of a pathogenic microbe which has been genetically modified such that at least one repressor gene for a heat shock protein is inactivated, thereby allowing constitutive expression of heat shock proteins, as a vaccine vector which further allows the expression of a heterologous antigen.

Preferably the pathogenic microbe is a prokaryotic organism, which is suitable for use as a vaccine vector.

Preferably the prokaryotic organism is BCG and the heterologous antigen fragment is the tetanus toxoid fragment C.

Alternatively the prokaryotic organism is Salmonella or Lactococcus, such that the vaccine can be administered orally.

Further described herein is the use of a pathogenic microbe which has been genetically modified such that at least one repressor gene for a heat shock protein is inactivated, thereby allowing constitutive expression of heat shock proteins, as a source of heat shock protein - peptide complexes for use in subunit and multi-subunit vaccines.

By "microbial pathogen" it is meant any pathogen capable of inducing an infectious disease in an animal, including particularly bacterial, protozoan, fungal or parasitic organisms.

By "stress-inducing stimuli" it is meant any stimuli that induces the production of SPs and in particular hsps in microbial pathogens, including heat or osmotic stress. Such stress-inducing stimuli also includes genetic changes designed to render constitutive the expression of stress proteins and in particular hsps in microbial pathogens. These genetic changes include the inactivation of repressor (suppressor) genes that function in the suppression of stress proteins and in particular the suppression of the genes which express heat shock proteins. This includes in particular the inactivation of the hspR suppressor gene, the stress gene regulatory protein genes MerR and HmrR, or the transcriptional control genes sigma and rho.

It will be appreciated that such genetic changes are readily achieved by molecular genetic means and include insertional mutagenesis using phage or transposon vectors, see for example Bucca et al. Mol.Microbiol. (1997) 17:633. It will also be appreciated that the presence of the genes for the operons, suppressor genes and SPs and hsps in microbial pathogens can be simply established by recombinant DNA techniques, see Current Techniques in Molecular Biology, (1999) Wiley Press.

Thus it should be appreciated that any microbe expressing these genes can be genetically modified to provide a vaccine according to this invention. This includes existing microbial organisms used as live or killed vaccines, such as *Mycobacteria, Salmonella, Vibrio and Listeria* and in particular attenuated varieties of these pathogens. In particular, Mycobacteria mutants, which have been genetically modified to delete the heat shock protein repressor gene could be used in a vaccine against tuberculosis, while Salmonella mutants which have been genetically modified to delete the heat shock protein repressor genes could be used in a vaccine against typhoid. The use of such organisms as vaccine vectors for the expression of heterologous genes is further provided by the present invention.

The term "vaccine" is used herein to denote to any composition containing an immunogenic determinant which stimulates the immune system such that it can better respond to subsequent infections. It will be appreciated that a vaccine usually contains an immunogenic determinant and an adjuvant, the adjuvant serving to non-specifically enhance the immune response to that immunogenic determinant. Suitable adjuvants are readily apparent to the person skilled in the art, and include; Freund's complete adjuvant, Freund's incomplete adjuvant, Quil A, Detox, ISCOMs or squalene.

However, it will be appreciated that the vaccine of the present invention may also be effective without an adjuvant.

For the non-genetic induction of SPs, the optimum conditions for inducing the SPs can readily be determined by simple trial and error with the effect of a change of stimuli being assessed using conventional techniques, such as in vivo testing on animals, or by other techniques, for example those described in 'Current Protocols in Immunology', Wiley Interscience, 1997. Other such conditions are described in PCT Application No GB00/03228 and citations referred to therein.

Further described herein is a method for exposing an animal to a vaccine of the invention by administering a pharmaceutically acceptable quantity of the vaccine of the invention, optionally in combination with an adjuvant, sufficient to elicit an immune response in the animal.

The animal is typically a human. However, the invention can also be applied to the treatment of other mammals such as horses, cattle, goats, sheep or swine, and to the treatment of birds, notably poultry such as chicken or turkeys. Preferably the microbial pathogen selected for use in a particular vaccine of the present invention causes disease or infection in the species of animal to which the vaccine is administered to, or a closely related species. The vaccines of this invention may be used as both prophylactic or therapeutic vaccines though it will be appreciated that they will be particularly useful as prophylactic vaccines due to their economy of production.

The vaccine compositions of the present invention may be administered by any suitable means, such as orally, by inhalation, transdermally or by injection and in any suitable carrier medium. However, it is preferred to administer the vaccine as an aqueous composition by injection using any suitable needle or needle-less technique.

It will be appreciated that the vaccine of the invention may be applied as an initial treatment followed by one or more subsequent "booster" treatments at the same or a different dosage rate at an interval of from 1 to 26 weeks between each treatment to provide prolonged immunisation against the pathogen.

The present invention will now be described, by way of example, with reference to the accompanying figures, wherein;
Figure 1 shows the nucleotide sequence of the hspR gene of *M. tuberculosis*, and
Figure 2 shows a list of identified suppressor genes which have homology to hspR.

### Example 1: Preparation of heat-induced microbes.

*M.vaccae* strain NCTC11659 was grown to saturation in Sauton's media, diluted into fresh media and grown overnight to give a log phase culture which was then heat-shocked at 42°C for 3 hours or at 39°C for 5 hours and cultured overnight. The cells were then washed in media, followed by a wash in saline and either lyophilised in individual vaccine aliquots or used directly for the immunisation of test animals. For comparison isolated endogeneous SP-peptide complexes were prepared as described in PCT Publication No WO 2001/013944. Essentially, washed stress-induced cells are then re-suspended in homogenisation buffer such as PBS with 0.5% Tween and cells are then disrupted by freeze-thaw cycles or using cell disruptor (e.g. bead beater, French press). The cell lysate is then treated by centrifugation, typically 3-5000g for 5 minutes, to remove the nuclei and cell debris, followed by a high speed centrifugation step, typically 100,000g for 15-30 minutes. The supernatant thus obtained is processed to give an SP/antigenic peptide fragment complex suitable for use in a vaccine. This can be done simply by ammonium sulphate precipitation which uses a 20-70% ammonium sulphate cut. Specifically, 20% (w/w) ammonium sulphate is added at 4°C, the precipitate is discarded, followed by the addition of more ammonium sulphate to bring the concentration to 70% w/w. The protein precipitate is harvested by centrifugation and then dialysed into an appropriate physiological, injectable buffer, such as saline, to remove the ammonium sulphate before use. The SP complexes may be used at any suitable concentration to provide the immunogenic determinant in the vaccine composition.

It is preferred that the amount of the induced stress protein-peptide complex is in the range of 10-600 µg, more preferably 10-100 µg, and most preferably 25 µg per kg of animal body weight.

In order to determine the immunogenicity of the SP complexes, T cell proliferation assays may be used. Suitable assays include the mixed-lymphocyte reaction (MLR), assayed by tritiated thymidine uptake, and cytotoxicity assays to determine the release of ⁵¹Cr from target cells, see 'Current Protocols in Immunology', Wiley Interscience, 1997. For Mycobacteria, MLRs can also be assayed for the induction of cytokine production such as the production of interferon gamma using the commercial kit (CSL Ltd). Alternatively, antibody production may be examined, using standard immunoassays or plaque-lysis assays, or assessed by intrauterine protection of a foetus, see 'Current Protocols in Immunology'.

Mice were immunised with either heat-stressed organisms or the endogenous SP-peptide complexes isolated from the heat stressed organisms in phosphate buffered saline without any added adjuvant in either the primary or booster vaccinations.

Immunisation with whole stress-induced organisms and in particular lyophilised organisms gave significantly better immunity than that induced by isolated endogeneous SP-peptide complexes including increased IFN-gamma and antibody production.

### Example 2: Preparation and use of constitutive hsp mutants.

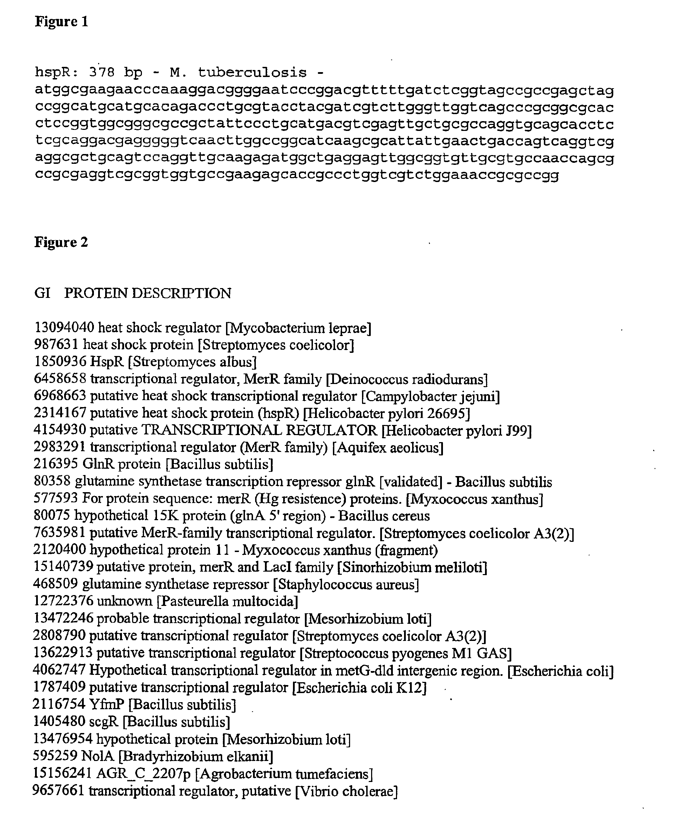

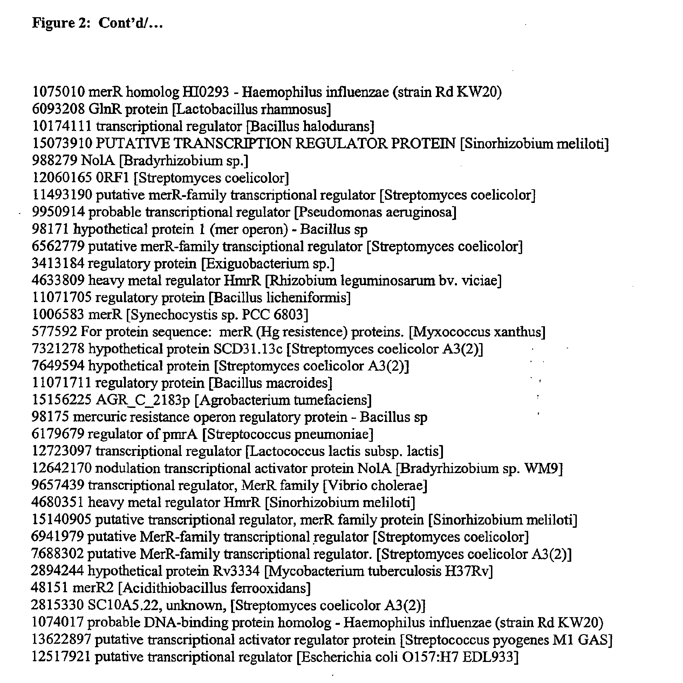

Constitutive hsp producing microbes can be constructed through the knockout of transcriptional regulator suppressor genes such as hspR, MerR (mercuric resistance operon regulatory protein), HmrR (heavy metal regulatory protein) and their homologues or the transcriptional control genes rho and sigma. Such genes are readily identified by screening of genome sequence databases with typical test sequences. An example of such a typical test sequence is the hspR gene of *M. tuberculosis* (shown in Figure 1). An example of a list of identified suppressors which have homology to the hspR gene is shown in Figure 2.

*M.bovis* strains constitutively producing hspCs were constructed by genetically engineering the *M. bovis* strain BCG to yield deletion mutants for the hspR gene. The hspR gene was deleted by homologous recombination using a suicide vector carrying a large fragment of the hspR gene and a kanamycin selection marker. The hspR gene fragment was cloned by PCR from BCG genomic DNA using primers derived from the *M. tuberculosis* hspR sequence shown in Figure 1. Such an approach should be widely applicable to the production of similar mutants from any prokaryote carrying the relevant suppressor gene.

BCG hspR deletion mutants were used to produce hspCs and the protein yields obtained from these were comparable to those obtained from heat shocked wild type strains. Such an approach should again.be widely applicable to the production of hspCs from any prokaryote carrying the relevant suppressor gene. These would provide a valuable resource for the manufacture of hspC-based vaccines. Thus hspCs produced from BCG hspR deletion mutants induced protective immunity against aerosol challenge by *M. tuberculosis* comparable to that induced by hspCs obtained from heat shocked wild type BCG strains.

BCG hspR deletion mutants can be also be used as vaccine vectors to express heterologous antigens. For example, mice immunised with a BCG hspR deletion mutant expressing the tetanus toxoid (TT) fragment C showed the production of anti-TT antibodies as detected by Western blotting. Such an approach should be widely applicable to the production of similar vectors for the expression of heterologous vaccine antigens using mutants from any prokaryote suitable for use as a vaccine vector. For example the use of *Salmonella* or *Lactococcus* mutants would enable the production of vectors targeted for mucosal delivery of vaccines. Vaccines developed on this principle would be particularly advantageous in that they could be administered orally.

## Claims

1. A vaccine composition comprising a killed, dried or attenuated bacterial pathogen as the immunogenic determinant, wherein the bacterial pathogen has been subjected to a stress inducing stimuli wherein the stress inducing stimuli is the genetic modification of the bacterial pathogen such that at least one repressor gene for a heat shock protein gene is inactivated, thereby allowing the constitutive expression of heat shock proteins.

2. A vaccine composition as claimed in claim 1 wherein the genetic modification results in the inactivation of the hspR repressor gene.

3. A vaccine composition as claimed in claim 1 wherein the genetic modification results in the inactivation of the stress gene regulatory protein genes MerR or HmrR.

4. A vaccine composition as claimed in claim 1 wherein the genetic modification results in the inactivation of the transcription control genes rho or sigma.

5. A vaccine composition as claimed in any of the preceding claims wherein the bacterial pathogen is selected from the group consisting of Mycobacteria, Salmonella, Vibrio, Listeria, Streptomyces, Helicobacter and Lactococcus.

6. A vaccine composition as claimed in any preceding claim, wherein the vaccine further comprises an adjuvant.

7. A vaccine composition as claimed in claim 6, wherein the adjuvant is selected from the group consisting of; Freund's complete adjuvant, Freund's incomplete adjuvant, Quil A, Detox, ISCOMs and squalene.

8. A vaccine composition as claimed in any of the preceding claims, wherein the vaccine is suitable for administration by injection.

9. A vaccine composition as claimed in claims 1 to 8 wherein the vaccine composition is suitable for oral administration.

10. A vaccine composition as claimed in claim 9, wherein the vaccine composition is suitable for delivery by means of a needle-less delivery format.

11. A vaccine composition as claimed in any one of claims 1 to 10 for use in the treatment of infection with a bacterial pathogen which is provided as the immunogenic determinant in the vaccine composition.

12. A composition as claimed in claim 11 wherein the vaccine is a prophylactic vaccine.

13. A composition as claimed in claim 11 wherein the vaccine is a therapeutic vaccine.

14. A composition as claimed in claim 11 wherein the vaccine is administered by injection.

15. A composition as claimed in claim 11 wherein the vaccine is administered by needle-less delivery.

16. A composition as claimed in claim 11 wherein the vaccine is administered transdermally.

17. A composition as claimed in claim 11 wherein the vaccine is administered by pulmonary delivery.

18. A composition as claimed in claim 11 wherein the vaccine is administered orally.

19. A method of producing a vaccine composition comprising an immunogenic determinant **characterised in that** said method comprises the steps of:
subjecting a bacterial pathogen to a stress inducing stimuli **characterised in that** the stress inducing stimuli is the inactivation of a gene which represses the expression of heat shock protein genes; and
using the stressed bacterial pathogen as the immunogenic determinant in the vaccine composition.

20. A method as claimed in claim 19, **characterised in that** the repressor gene is the hspR gene.

21. A method as claimed in claim 19, **characterised in that** the repressor gene is MerR or HmrR.

22. A method as claimed in any of claims 19 to 21 wherein the bacterial pathogen cells are killed prior to use in said vaccine.

23. A method as claimed in any of claims 19 to 21 wherein the bacterial pathogen cells are dried prior to use in said vaccine.

## Patentansprüche

1. Eine lmpfstoffzusammensetzung, die einen abgetöteten, getrockneten oder abgeschwächten bakteriellen Krankheitserreger als die immunogene Determinante beinhaltet, wobei der bakterielle Krankheitserreger einem Stress induzierenden Stimulus ausgesetzt wurde, wobei der Stress induzierende Stimulus die genetische Veränderung des bakteriellen Krankheitserregers ist, so dass mindestens ein Repressorgen für ein Hitzeschockproteingen inaktiviert wird, wodurch die konstitutive Expression von Hitzeschockproteinen ermöglicht wird.

2. lmpfstoffzusammensetzung gemäß Anspruch 1, wobei die genetische Veränderung in der Inaktivierung des hspR-Repressorgens resultiert.

3. lmpfstoffzusammensetzung gemäß Anspruch 1, wobei die genetische Veränderung in der Inaktivierung der Stressgen-Regulatorproteingene MerR oder HmrR resultiert.

4. lmpfstoffzusammensetzung gemäß Anspruch 1, wobei die genetische Veränderung in der Inaktivierung der Transkriptionskontrollgene Rho oder Sigma resultiert.

5. Impfstoffzusammensetzung gemäß einem der vorhergehenden Ansprüche, wobei der bakterielle Krankheitserreger aus der Gruppe, die aus Mykobakterien, Salmonella, Vibrio, Listeria, Streptomyces, Helicobacter und Lactococcus besteht, ausgewählt ist.

6. lmpfstoffzusammensetzung gemäß einem der vorhergehenden Ansprüche, wobei der Impfstoff ferner ein Adjuvans beinhaltet.

7. lmpfstoffzusammensetzung gemäß Anspruch 6, wobei das Adjuvans aus der Gruppe, die aus komplettem Freund-Adjuvans, inkomplettem Freund-Adjuvans, Quil A, Detox, ISCOMs und Squalen besteht, ausgewählt ist.

8. lmpfstoffzusammensetzung gemäß einem der vorhergehenden Ansprüche, wobei der Impfstoff zur Verabreichung durch Injektion geeignet ist.

9. Impfstoffzusammensetzung gemäß den Ansprüchen 1 bis 8, wobei die Impfstoffzusammensetzung zur oralen Verabreichung geeignet ist.

10. lmpfstoffzusammensetzung gemäß Anspruch 9, wobei die Impfstoffzusammensetzung zur Zuführung mittels eines nadellosen Zuführformats geeignet ist.

11. Impfstoffzusammensetzung gemäß einem der Ansprüche 1 bis 10 zur Verwendung bei der Behandlung einer Infektion mit einem bakteriellen Krankheitserreger, der als die immunogene Determinante in der Impfstoffzusammensetzung bereitgestellt ist.

12. Zusammensetzung gemäß Anspruch 11, wobei der Impfstoff ein prophylaktischer Impfstoff ist.

13. Zusammensetzung gemäß Anspruch 11, wobei der Impfstoff ein therapeutischer Impfstoff ist.

14. Zusammensetzung gemäß Anspruch 11, wobei der Impfstoff durch Injektion verabreicht wird.

15. Zusammensetzung gemäß Anspruch 11, wobei der Impfstoff durch nadellose Zuführung verabreicht wird.

16. Zusammensetzung gemäß Anspruch 11, wobei der Impfstoff transdermal verabreicht wird.

17. Zusammensetzung gemäß Anspruch 11, wobei der Impfstoff durch pulmonale Zuführung verabreicht wird.

18. Zusammensetzung gemäß Anspruch 11, wobei der Impfstoff oral verabreicht wird.

19. Ein Verfahren zum Herstellen einer lmpfstoffzusammensetzung, die eine immunogene Determinante beinhaltet, **dadurch gekennzeichnet, dass** das Verfahren die folgenden Schritte beinhaltet:
Aussetzen eines bakteriellen Krankheitserregers gegenüber einem Stress induzierenden Stimulus, **dadurch gekennzeichnet, dass** der Stress induzierende Stimulus die Inaktivierung eines Gens ist, das die Expression von Hitzeschockproteingenen unterdrückt; und
Verwenden des gestressten bakteriellen Krankheitserregers als die immunogene Determinante in der lmpfstoffzusammensetzung.

20. Verfahren gemäß Anspruch 19, **dadurch gekennzeichnet, dass** das Repressorgen das hspR-Gen ist.

21. Verfahren gemäß Anspruch 19, **dadurch gekennzeichnet, dass** das Repressorgen MerR oder HmrR ist.

22. Verfahren gemäß einem der Ansprüche 19 bis 21, wobei die bakteriellen Krankheitserregerzellen vor der Verwendung in dem Impfstoff abgetötet werden.

23. Verfahren gemäß einem der Ansprüche 19 bis 21, wobei die bakteriellen Krankheitserregerzellen vor der Verwendung in dem Impfstoff getrocknet werden.

## Revendications

1. Une composition de vaccin comprenant un pathogène bactérien tué, desséché ou atténué en tant que déterminant immunogène, où le pathogène bactérien a été soumis à un stimulus inducteur de stress où le stimulus inducteur de stress est la modification génétique du pathogène bactérien de façon à ce qu'au moins un gène répresseur pour un gène de protéine de choc thermique soit inactivé, permettant de ce fait l'expression constitutive de protéines de choc thermique.

2. Une composition de vaccin telle que revendiquée dans la revendication 1 où la modification génétique a pour résultat l'inactivation du gène répresseur hspR.

3. Une composition de vaccin telle que revendiquée dans la revendication 1 où la modification génétique a pour résultat l'inactivation des gènes de protéines régulatrices du gène de stress MerR ou HmrR.

4. Une composition de vaccin telle que revendiquée dans la revendication 1 où la modification génétique à pour résultat l'inactivation des gènes de contrôle de transcription rho ou sigma.

5. Une composition de vaccin telle que revendiquée dans n'importe lesquelles des revendications précédentes où le pathogène bactérien est sélectionné dans le groupe consistant en Mycobactéries, Salmonelle, Vibrio, Listeria, Streptomyces, Helicobacter et Lactococcus.

6. Une composition de vaccin telle que revendiquée dans n'importe quelle revendication précédente, où le vaccin comprend de plus un adjuvant.

7. Une composition de vaccin telle que revendiquée dans la revendication 6, où l'adjuvant est sélectionné dans le groupe consistant en : adjuvant complet de Freund, adjuvant incomplet de Freund, Quil A, Detox, ISCOMs et squalène.

8. Une composition de vaccin telle que revendiquée dans n'importe lesquelles des revendications précédentes, où le vaccin convient à l'administration par injection.

9. Une composition de vaccin telle que revendiquée dans les revendications 1 à 8 où la composition de vaccin convient à l'administration orale.

10. Une composition de vaccin telle que revendiquée dans la revendication 9, où la composition de vaccin convient à l'apport par le biais d'un format d'apport sans aiguille.

11. Une composition de vaccin telle que revendiquée dans n'importe laquelle des revendications 1 à 10 destinée à être utilisée dans le traitement d'infection avec un pathogène bactérien qui est fourni en tant que déterminant immunogène dans la composition de vaccin.

12. Une composition telle que revendiquée dans la revendication 11 où le vaccin est un vaccin prophylactique.

13. Une composition telle que revendiquée dans la revendication 11 où le vaccin est un vaccin thérapeutique.

14. Une composition telle que revendiquée dans la revendication 11 où le vaccin est administré par injection.

15. Une composition telle que revendiquée dans la revendication 11 où le vaccin est administré par apport sans aiguille.

16. Une composition telle que revendiquée dans la revendication 11 où le vaccin est administré par voie transdermique.

17. Une composition telle que revendiquée dans la revendication 11 où le vaccin est administré par voie pulmonaire.

18. Une composition telle que revendiquée dans la revendication 11 où le vaccin est administré par voie orale.

19. Une méthode pour produire une composition de vaccin comprenant un déterminant immunogène **caractérisée en ce que** ladite méthode comprend les étapes de :
soumettre un pathogène bactérien à un stimulus inducteur de stress **caractérisée en ce que** le stimulus inducteur de stress est l'inactivation d'un gène qui réprime l'expression de gènes de protéines de choc thermique ; et
utiliser le pathogène bactérien stressé en tant que déterminant immunogène dans la composition de vaccin.

20. Une méthode telle que revendiquée dans la revendication 19, **caractérisée en ce que** le gène répresseur est le gène hspR.

21. Une méthode telle que revendiquée dans la revendication 19, **caractérisée en ce que** le gène répresseur est MerR ou HmrR.

22. Une méthode telle que revendiquée dans n'importe lesquelles des revendications 19 à 21 où les cellules bactériennes pathogènes sont tuées avant d'être utilisées dans ledit vaccin.

23. Une méthode telle que revendiquée dans n'importe lesquelles des revendications 19 à 21 où les cellules bactériennes pathogènes sont desséchées avant d'être utilisées dans ledit vaccin.
